# EUROPEAN PATENT SPECIFICATION

(11) **EP 4 061 481 B1**
(45) Date of publication and mention of the grant of the patent: **01.01.2025**
(21) Application number: 19953199.7
(22) Date of filing: 06.12.2019
(51) Int. Cl.: A61N 2/02, A61N 2/00

(54) **CLINICAL AND PERSONAL ELECTROMAGNETIC STIMULATOR AND IONTOPHORESIS DEVICE FOR TREATING RETINA AND OPTICAL NERVE DISEASES**
KLINISCHER UND PERSÖNLICHER ELEKTROMAGNETISCHER STIMULATOR UND IONTOPHORESEVORRICHTUNG ZUR BEHANDLUNG VON ERKRANKUNGEN DER NETZHAUT UND DES SEHNERVS
DISPOSITIF CLINIQUE ET PERSONNEL DE STIMULATION ÉLECTROMAGNÉTIQUE À IONOPHORÈSE POUR LE TRAITEMENT DES MALADIES DE LA RÉTINE ET DU NERF OPTIQUE

(30) Priority: 19.11.2019 TR 201918027
(43) Date of publication of application: 28.09.2022
(73) Proprietor: Bioretina Biyoteknoloji Ve Biyomedikal Saglik Yatirimlari Anonim Sirketi, Gölbasi/Ankara (TR)
(72) Inventor: ARSLAN, Umut, Gölba /Ankara (TR); ÖZMERT, Emin, Gölba /Ankara (TR)
(74) Representative: Araujo, Daniel
(86) International application number: PCT/TR2019/000086
(87) International publication number: WO 2021/101459

(56) References cited:
- WO-A1-2019/173866
- CN-A- 103 126 863
- CN-A- 103 211 676
- CN-A- 103 211 676
- US-A1- 2005 137 649
- US-A1- 2019 143 116
- ÖZMERT EMIN ET AL: "Management of Deep Retinal Capillary Ischemia by Electromagnetic Stimulation and Platelet-Rich Plasma: Preliminary Clinical Results", ADVANCES IN THERAPY, HEALTH COMMUNICATIONS, METUCHEN, NJ, US, vol. 36, no. 9, 5 August 2019 (2019-08-05), pages 2273 - 2286, XP036861019, ISSN: 0741-238X, [retrieved on 20190805], DOI: 10.1007/S12325-019-01040-2
- EMIN OZMERT ET AL.: "Management of Deep Retinal Capillary Ischemia by Electromagnetic Stimulation and Platelet-Rich Plasma : Preliminary Clinical Results", ADVANCES IN THERAPY, vol. 36, 5 August 2019 (2019-08-05), pages 2273 - 2286, XP036861019, DOI: 10.1007/ s 12325-019 -01040-2

## Description

### Technical Field

The invention is related to electromagnetic stimulator and iontophoresis devices used for treating retina and optical nerve diseases.

The invention is particularly related to a personal electromagnetic stimulator and iontophoresis device which enables to reach the required threshold values for treating retina and optical nerve diseases with very low magnetic fields, which eliminates thermal damage risk formed at the cellular level by means of cooling cages, enables the transfer of ocular drug injections made to the retina using the electromagnetic iontophoresis method and allows usage by the patient alone at home, who has low vision, without receiving any professional help, thanks to the audio command guidance system.

### Prior Art

Chronic retina and optical nerve diseases can be ischemic or degenerative. In degenerative diseases, the synthesis of the growth factors which enable vital functions of retina nerve cells are damaged genetically. In ischemic patients however, the blood flow and supply to retina nerve cells are damaged. In both cases, chronic and progressive visual loss is experienced and in the end legal blindness occurs.

The stimulation of the retina and optical nerve electromagnetically leads to retina blood flow increase at a cellular level, increase in vascular permeability and increase of receptor sensitivity in growth factors. Simultaneous stimulation of the occipital cortex leads to an increase in neurotrophic growth factor levels in the retina. The simultaneous electromagnetic stimulation of the retina, optical nerve, optical tracts and visual cortex leads to cellular depolarization and phosphene formation. In order for all of these effects to be realized a suprathreshold electromagnetic field is required. The required suprathreshold magnetic field can be obtained by the correct positioning of electromagnetic coils, correct frequency, power, intensity and time. These effects that are reparative in retina and optical nerve diseases must be applied at certain periods and times mostly for a lifelong period in chronic diseases. Due to this reason, in order to obtain an efficient result, the patient needs to use an electromagnetic stimulator on his/her own at home continuously and correctly.

In order for electromagnetic stimulation to be effective in retina and optical nerve diseases, the occipital cortex, inter-hemispherical sulcus, and the retina and optical nerves need to be stimulated at the same time. Retina and optical nerve stimulation is carried out from a vertex distance which is the most efficient distance. The vertex distance, is the closest distance to the cornea apex which does not prevent the closing of the eyelids. However the electromagnetic stimulator devices in the prior art, may cause thermal damage at a cellular level and therefore it is not possible to stimulate the eyeball, retina and optical nerve from the vertex distance. At the same time the devices of the prior art are suitable to be used under the supervision of a health personnel. The incorrect usage applied under the supervision of people who are not experts, may lead to many complications including visual loss. Some drug injections are applied in treating retina diseases. In order for these drugs to reach the retina, an iontophoresis procedure is required. In order for an iontophoresis procedure to be electromagnetically efficient the coils need to be effective simultaneously, in the same way, in the correct position and at the correct distance. In order to establish correct stimulation points and correct electrical polarization, the device needs to have a standard design. The present iontophoresis devices have effect not with an electromagnetic field but with an electric current. Electromagnetic iontophoresis is designed such that it does not contact the eye and therefore it is much safer than electrical iontophoresis devices.

The document numbered US2017165485 can be given as an example to the stimulator device in literature. In said document a device that has been developed to treat visual complaints during headaches and migraines is described. It is not possible to stimulate the eyeball, retina and optical nerves from the vertex distance with said device, it is possible to stimulate the supraorbital region at most with this device. It is not possible to reach effective suprathreshold values in the eye, during the treatment of retina and optical nerves with this device that is not indicated for treating degenerative and ischemic retina optical nerve diseases. The device does not have any cooling mechanism in order for the device to be used from the vertex distance. The device does not have a standard cap system placement design to the correct poles in order to perform electromagnetic iontophoresis. This design that has been defined with general terms according to requirements for placement of electromagnetic coils and electrodes does not have the required standard design in order to perform iontophoresis. This device does not comprise a design having the standard shape, number, distance and parameters that can apply an electromagnetic field at the vertex distance, and to establish standard poles between other electromagnetic coils.

Another example is the document numbered WO2009063435. Said document is related to a sensor system which shows if the coils have been placed in the correct position on the occipital cortex during amblyopia treatment. It is not indicated for being used in treating retina and optical nerve diseases. The device does not have any cooling mechanism in order for the device to be used from the vertex distance. The device does not have a standard cap system placement design to the correct poles in order to perform electromagnetic iontophoresis. Therefore said system is not designed to stimulate the eyeball, retina and optical nerves from the vertex distance.

Another example is the document numbered US2009099623. Said document is related to the treatment of medical conditions associated with the central nervous system and to increasing cognitive functions. The device does not have any cooling mechanism in order for the device to be used from the vertex distance. The device does not have a standard cap system placement design to the correct poles in order to perform electromagnetic iontophoresis. Said device is not designed or indicated for treating degenerative and ischemic retina optical nerve diseases.

Another example is the document numbered US2013137918. Said document is related to a low frequency magnetic stimulation treatment device. The device does not have any cooling mechanism in order for the device to be used from the vertex distance. The device does not have a standard cap system placement design to the correct poles in order to perform electromagnetic iontophoresis. Said device is not designed or indicated for treating degenerative and ischemic retina optical nerve diseases.

Another example is the document numbered GB2295093. Said document is related to a synchronized electromagnetic therapy device. The device does not have any cooling mechanism in order for the device to be used from the vertex distance. The device does not have a standard cap system placement design to the correct poles in order to perform electromagnetic iontophoresis. Said device is not designed or indicated for treating degenerative and ischemic retina optical nerve diseases.

Document CN103211676 describes a electromagnetic stimulation instrument for the eye but lacks an appropriate cooling mechanism.

As a result, the presence of the above mentioned problems and the inadequacy of the present solutions has made it a necessity to provide a development in the related technical field.

### Aims of the Invention

The present invention is related to a personal electromagnetic stimulator and iontophoresis device for treating retina and optical nerve diseases which eliminate the above mentioned disadvantages and bring about new advantages.

The aim of the invention is reach the required threshold values with very low magnetic fields in order to treat retina and optical nerve diseases by means of the cap worn by the patient, comprising electromagnetic coils that has a design which can be correctly positioned and which can stimulate the eyeball, retina and optical nerves directly.

The aim of the invention is to eliminate the risk of thermal damage at the cellular level as the electromagnetic coils are covered with cooling cages.

Another aim of the invention is to increase the passage of the ocular drug injections to the retina by means of the electromagnetic iontophoresis method as the electromagnetic coils are connected to the cap system such that it allows correct polarization.

Another aim of the invention is to prevent the formation of opacities in the cornea, lens and vitreous and to prevent permanent visual loss by eliminating thermal damage risk at the cellular level.

Another aim of the invention is to enable correct, safe and efficient usage of the stimulator device by the patient alone, without requiring professional assistance by means of the audio commands provided in different languages, to the patient having visual impairment.

Another aim of the invention is to notify the doctor following up the patient if the patient is using the device correctly or not, by means of the counter system on the device.

The invention is related to a stimulator and iontophoresis device used for treating retina and optical nerve diseases, in order to carry out all of the aims that can be construed from the information provided above and the detailed description below. The invention is characterized in that it comprises;
- a cap made of two parts; one being the front cap (10a) comprising an electromagnetic coil assembly including electromagnetic coils which create 4 electromagnetic fields at a vertex distance to the eyeballs, that have been covered with a cooling cage, and the other being the rear cap comprising an electromagnetic coil assembly including electromagnetic coils which create 4 electromagnetic fields that stimulate the occipital cortex region and 1 electromagnetic field that stimulates the inter-hemispheric region, that have been covered with a cooling cage,
- wherein said device comprises a cooling compressor connected with cooling pipes that pass through the connection pipe that is connected with the cooling cages inside the front cap and rear cap and gas perfluorocarbon tank, an electronic card and a processor having a counter thereon which adjusts the intensity, frequency and time of the electromagnetic field created by the electromagnetic coils, an electronic control box having a speaker connected to the electronic card and processor.

The structural and characteristic features of the invention and all of its advantages can be better understood by means of the detailed description and by referring to the attached figures. Due to this reason, if an evaluation needs to be carried out, it must be done so by taking into consideration the attached figures and the detailed description.

### Figures Describing the Invention

**Figure 1****:** General view of the electromagnetic iontophoresis device subject to the invention.
**Figure 2****:** The perspective view of the internal detail of the stimulator and iontophoresis device cap subject to the invention.
**Figure 3****:** The perspective view of the stimulator and iontophoresis device cap subject to the invention.
**Figure 4****:** Detailed view of the electromagnetic coil assembly of the stimulator and iontophoresis device subject to the invention.
**Figure 5****:** The detailed below of the stimulator and iontophoresis device cap and the electronic control box subject to the invention.

### Description of the Part Reference Numbers

**10.** Cap
**10a.** Front Cap
**10b.** Rear Cap
**11.** Connection rail
**12.** Electromagnetic coil assembly
**121.** Electromagnetic coil
**1211.** LED indicator
**1212.** Magnetic field sensor
**122.** Cooling cage
**1221.** Cooling tank
**1222.** Gas inlet hole
**1223.** Gas outlet hole
**123.** Electric cable
**124.** Cooling pipe
**13.** Sponge rubber
**20.** Electronic control box
**21.** Electronic card and processor
**211.** Counter
**22.** Cooling compressor
**23.** Gas perfluorocarbon tank
**24.** Screen
**25.** Speaker
**26.** Control panel
**27.** Power Supply
**28.** On/off button
**30.** Connection pipe

### A. Stimulator and iontophoresis device

### Detailed Description of the Invention

In this detailed description the preferred embodiments of the stimulator and iontophoresis device (A) subject to the invention has been described only in order to better explain the subject matter and such that it does not create any kind of limiting effect.

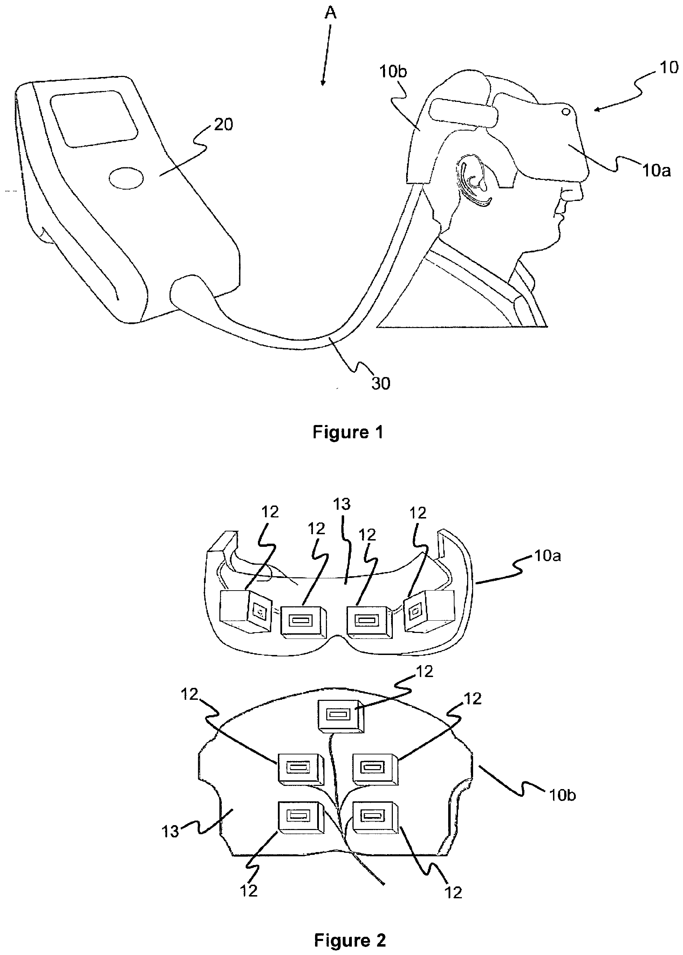

The general view of the stimulator and iontophoresis device (A) subject to the invention has been provided in Figure 1. The stimulator and iontophoresis device (A) according to the invention basically comprises; a cap (10) made of two parts, one being the front cap (10a) and the other being the rear cap (10b) connected to each other by a connection rail (11) having an electromagnetic coil assembly (12) which enables to reach the required threshold values in order to treat retina and optical nerve diseases with very low magnetic fields, and an electronic control box (20) connected via the connection pipe (30) to the cap (10) having electric cables (123) and cooling pipes (124) passing therein.

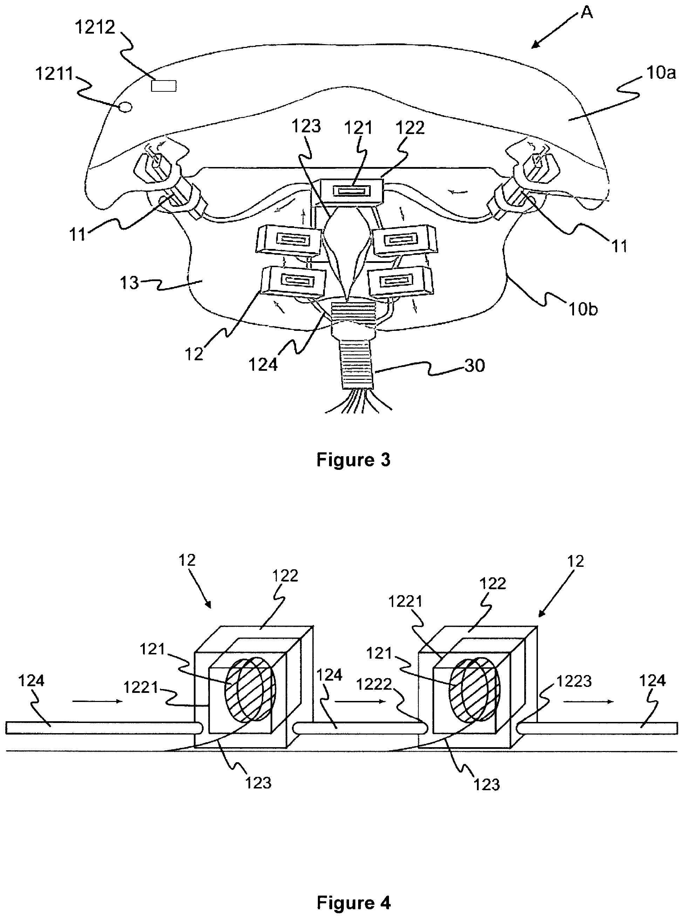

As it can be seen from figure 2 and 3, the cap (10) that is worn over the head of the patient is made of two parts being the front cap (10a) and the rear cap (10b) connected to each other with a connection rail (11) from their edges. Said front cap (10a) and the rear cap (10b) can be adjusted according to the diameter of the user by being brought closer or by being distanced apart via the connection rail (11). Therefore by means of the adjustable configuration of the cap (10) suitable positioning of the electromagnetic coil assembly (12) has been provided for all head diameters. The front cap (10a) and the rear cap (10b) is made of noninflammable, antibacterial, antistatic medical plastic, carbon fibre or any other kind of medical material.

The cap (10) comprises a total of 9 electromagnetic coil assemblies (12) that are fixedly embedded in order to reach the required threshold values with very low magnetic fields to treat retina and optical nerve diseases.

The front cap (10a) comprises 4 electromagnetic coil assemblies (12) that create electromagnetic fields at the vertex distance to the eyeballs and the rear cap (10b) comprises a total of 5 electromagnetic coil assemblies (12) that are in contact with the scalp wherein 4 electromagnetic fields stimulate the occipital cortex region and 1 electromagnetic field stimulates the inter-hemispheric region.

The sections besides the electromagnetic coil assembly (12) at the inner surfaces of the front cap (10a) and the rear cap (10b) are covered with a smart medical sponge rubber (13) that is noninflammable, biocompatible, antiallergic and antibacterial. Said smart sponge rubber (13) enables the electromagnetic coil assemblies (12) to remain in the correct position during usage.

The electromagnetic coil assembly (12) illustrated in Figure 4, is generally formed of an electromagnetic coil (121) and a cooling cage (122). Said electromagnetic coils (121) have polygonal form, which enables at least 2 electromagnetic coils (121), which stimulate an eyeball, to contact each other with a large surface area and which enables the two electromagnetic coils (121) that are in contact with each other to form a magnetic effective field that reaches the retina and optical nerve depth continuously from a vertex distance. The vertex distance is the distance which does not prevent the closing of the eyelids and which is the distance closest to the cornea apex. Therefore the electromagnetic coils (121) can affect the eyeball completely at the magnetic field depth without touching the eye. Therefore the placement of the electromagnetic coil assemblies (12) in the cap (10), the distance of the coils to each other, the polygonal structure and the intensity of the magnetic field allow the formation of suitable poles for iontophoresis. The arrangement of the electrical connections of the electromagnetic coils (121) are arranged in the front cap (10a) and the rear cap (10b) in order to form a standard and correct polarization on the cap (10); such that random placement is not allowed.

LED indicators (1211) and magnetic field sensors (1222) are provided on each of the electromagnetic coils (121). Therefore the tracking of active and passive electromagnetic coils (121) have been enabled. The magnetic field sensors (1222) enable the transmission of the magnetic field formed, when the electromagnetic coil (121) is active onto the screen (24) of the electronic control box (20). As a result when the stimulator device (A) is active, by which electromagnetic coil (121) the amount of magnetic field is created and if this field is correct or not can be tracked during the duration of the treatment process.

The electromagnetic coils (121) receive their power from the power supply (27) in the electronic control box (20) to which it is connected via electrical cables (123). Said power supply (27) can be an adapter and/or battery-accumulator that operates directly with the electric network. If the stimulator and iontophoresis device (A) is operated with electricity, the battery-accumulator steps in order not to delay the treatment time and the battery-accumulator percentage can be seen on the screen (27).

In order to prevent thermal damage risk at the cellular level that may be encountered due to electromagnetic coils (121), a cooling cage (122) has been covered over each electromagnetic coil (121). The cooling cages (122) are made of fibreglass, fibre carbon or any kind of other medical material, formed as two layered hollow impermeable boxes. Each cooling cage (122) has a cooling tank (1221) which allows gas circulation therein, and a gas outlet hole (1222) and a gas inlet hole (1223) allowing the entrance and discharging of the cooling gas. The cooling compressor (22) in the electronic control box (20) and the elastic cooling pipes (124) extending from the gas perfluorocarbon tank (23) pass through each cooling cage (122) and allow the gas to be pumped into the cooling tank (1221) via the gas inlet hole (1222). The cooling gas exiting out of the cooling tank (1221) via the gas outlet hole (1223) cools the next cooling tank (1221) and then returns to the cooling compressor (22) and the cooling tank (1221) by recirculation.

The electric cables (123) connected to the electromagnetic coils (121) have been isolated below the cooling cage (122) and the cooling pipes (124) used in the cooling process is isolated under sponge rubber (13). Although said electric cables (123) and cooling pipes (124) pass through the connection rails (11), they are formed as an accordion such that they can be extended or shortened. This ensures adjustment ease for each head diameter.

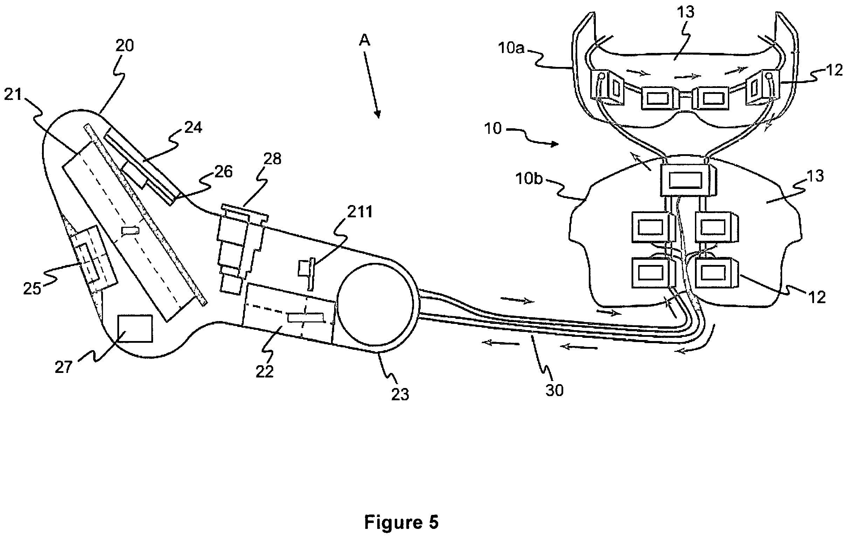

As it can be seen in Figure 5, the electronic control box (20) connected to the cap (10) via the electric cables (123) passing through the connection pipe (30), includes an electronic card and a processor (21) which adjusts the electromagnetic field intensity, frequency and time created by the electromagnetic coils (121). These parameters cannot be changed by the patient in order for the stimulator device (A) to be used safely and efficiently at home, alone by the patient. The patient can only control the on/off button (28). When the on/off button (28) is pressed, as standard, the stimulator device (A) creates 2000 milligauss electromagnetic field for 30 minutes at 42 hertz frequency from each electromagnetic coil (121). These parameters are the average suprathreshold values that are obtained following clinical trials. The determined frequency and electromagnetic field intensity in order to carry out effective electromagnetic iontophoresis, has been fixed and it cannot be changed by the patient.

In the case that the doctor following up the patient desires to conduct changes in the parameters with an aim besides performing electromagnetic iontophoresis, the technical staff can adjust the magnetic field intensity, frequency and time and which electromagnetic coil (121) needs to be passive-active. This adjustment is carried out by USB or WIRELESS connections or a password entered via the control panel (26) located inside the electronic control box (20).

By means of the counter (211) located on the electronic card and processor (21), the usage frequency of the stimulator device (A) by the patient and compatibility with treatment can be monitored by the doctor that is following up the patient.

The cooling compressor (20) and the gas perfluorocarbon tank (23) in the electronic control box (20) step in as soon as the stimulator device (A) creates an electromagnetic field and the electromagnetic coils (121) are cooled during the operation of the stimulator device (A) via the cooling cages (122) with +4°C gas perfluorocarbon circulation. This cooling degree can be changed via the control panel (26) by the technical staff following the request of the doctor. The patient cannot control the cooling system and its temperature.

The electronic control box (20) is configured with a speaker (25) connected to an electronic card and processor (21), wherein said speaker (25) enables to guide the patient with audio commands in order for the patient who has visual impairment to be able to use the stimulator and iontophoresis device (A) alone, safely, correctly and efficiently. Thereby the audio guidance of the patient is provided with the speaker (25) following the detection by the magnetic field sensors (1212) connected to the electronic card and processor (21) that determine if the front cap (10a) is at a vertex distance to the eyeball or not, and if the electromagnetic coil assemblies (12) at the rear cap (10b) are at the correct contact distance symmetrically to the occipital cortex and inter-hemispheric regions. At the same time the starting of the treatment, ending of the treatment and remaining time are vocally notified to the patient via the speaker (25). Additionally by means of the electronic card and processor (21) the patient with low vision can be guided with commands in different languages.

The required parameters of the stimulator and iontophoresis device (A) for therapy, is efficient with various setting adjustments and is suitable for safe usage in order to prevent incorrect usage.

The magnetic field intensity at the electromagnetic coils (121), frequency, time and the electromagnetic coil (121) that is active, number of usages, and activation information of the stimulator and iontophoresis device (A) are collected via the counter (211) and such information can be seen on the screen (24) that is included in the electronic control box (20).

The electric cables (123) leading to the electromagnetic coil (121) and exiting out of the electronic control box (20) and the cooling pipes (124) between the cooling compressor (22) and the cooling cage (122) are housed as a group inside the connection pipe (30) that is positioned between the cap (10) and the electronic control box (20) such that they are insulated and isolated.

## Claims

1. A stimulator and iontophoresis device (A) used for treating retina and optical nerve diseases, comprising:
- a cap (10) made of two parts; one being the front cap (10a) comprising an electromagnetic coil assembly (12) including electromagnetic coils (121) which create 4 electromagnetic fields at a vertex distance to the eyeballs, and cooling cages (122), each electromagnetic coil (121) being covered with a respective cooling cage, and the other being the rear cap (10b) also comprising an electromagnetic coil assembly (12) including electromagnetic coils (121) which create 4 electromagnetic fields that stimulate the occipital cortex region and 1 electromagnetic field that stimulates the inter-hemispheric region, and cooling cages (122), each electromagnetic coil (121) being covered with a respective cooling cage,
- the device also comprising an electronic control box (20), comprising: a cooling compressor (22), a gas perfluorocarbon tank (23), an electronic card and a processor (21) having a counter (211) thereon configured to adjust the intensity, frequency and time of the electromagnetic field created by the electromagnetic coils (121), and a speaker (25) connected to the electronic card and processor (21), wherein the cooling compressor (22) is connected with the cooling cages (122) inside the front cap (10a) and rear cap (10b) through cooling pipes (124) that are housed in a connection pipe (30).

2. A stimulator and iontophoresis device (A) according to claim 1, comprising a connection rail (11) which is located at the sides of the front cap (10a) and the rear cap (10b) in order to connect the front cap (10a) and rear cap (10b) to each other such as to extend or shorten the distance between the front and rear cap.

3. A stimulator and iontophoresis device (A) according to claim 1, wherein the front cap (10a) and the rear cap (10b) are made of noninflammable, antibacterial, antistatic medical plastic or carbon fibre.

4. A stimulator and iontophoresis device (A) according to claim 1, wherein the sections besides the electromagnetic coil assembly (12) at the inner surfaces of the front cap (10a) and the rear cap (10b), are covered with a smart sponge rubber (13).

5. A stimulator and iontophoresis device (A) according to claim 4, wherein said sponge rubber (13) is made of a medical, inflammable, biocompatible, antiallergic and antibacterial material.

6. A stimulator and iontophoresis device (A) according to claim 1, wherein the electromagnetic coil (121) is located inside the electromagnetic coil assembly (12) within said front cap (10a) and rear cap (10b) and has a polygonal form and is configured to create a 2000 milligauss magnetic field for 30 minutes at 42 hertz frequency.

7. A stimulator and iontophoresis device (A) according to claim 1 or 6, wherein said cooling cage (122) located on said electromagnetic coil (121), is configured to prevent the risk of thermal damage occurring at cellular levels on the eyeballs.

8. A stimulator and iontophoresis device (A) according to claim 1 or 7, wherein said cooling cage (122) is a double layered impermeable hollow box made of fibreglass, carbon fibre or any other medical material.

9. A stimulator and iontophoresis device (A) according to claim 1 or 7, wherein said cooling cage (122) has a cooling tank (1221) which allows gas circulation therein, and a gas outlet hole (1222) and a gas inlet hole (1223) allowing the entrance and discharging of the cooling gas.

10. A stimulator and iontophoresis device (A) according to claim 1, wherein said cooling pipes (124) are configured to enable recirculation between the cooling tank (1221) and the cooling compressor included in the cooling cage (122).

11. A stimulator and iontophoresis device (A) according to claim 1, comprising a control panel (26) located in the electronic control box (20) which enables to adjust the intensity, frequency and time and the active-passive state of the electromagnetic coils (121) via USB or WIRELESS connections and at the same time, enables to adjust the cooling degree of the cooling gas in the cooling cages (122).

12. A stimulator and iontophoresis device (A) according to claim 1, wherein said counter (211) located in the electronic control box (20) is configured to enable the doctor following up the patient to monitor the treatment compatibility of the patient and the frequency of usage of the stimulator and iontophoresis device (A) by the patient.

13. A stimulator and iontophoresis device (A) according to claim 1, wherein said speaker (25) connected to said electronic card and processor (21), enables to guide the patient with audio commands in different languages.

14. A stimulator and iontophoresis device (A) according to claim 1 or 6, comprising a LED indicator (1211) located on said electromagnetic coil (121) which allows to track the active and passive electromagnetic coils (121).

15. A stimulator and iontophoresis device (A) according to claim 1 or 6, comprising magnetic sensors (1212) located on the electromagnetic coil (121) which reflect the magnetic field created when the electromagnetic coil (121) is active, onto the screen (24) included in the electronic control box (20) and at the same time which determine if the electromagnetic coil assemblies (12) are at the correct symmetrical contact distance to the patient.

16. A stimulator and iontophoresis device (A) according to claim 1 comprising a screen (24) that is located on the electronic control box (20), which reflects via the counter (211) the information that is collected, such as the magnetic field intensity of the electromagnetic coils (121), frequency, time and the electromagnetic coil (121) that is active, number of usages, and activation information.

17. A stimulator and iontophoresis device (A) according to claim 1, wherein said connection pipe (30) is positioned between the cap (10) and the electronic control box (20) and houses as a group, the electric cables (123) leading to the electromagnetic coil (121) and exiting out of the electronic control box (20) and the cooling pipes (124) between the cooling compressor (22) and the cooling cage (122) such that they are insulated and isolated.

18. A stimulator and iontophoresis device (A) according to claim 1, comprising a power supply (27) located inside the electronic control box (20), which is a battery-accumulator and/or adapter operating directly with the electricity network, wherein said power supply is connected to the electromagnetic coils (121) via electric cables (123).

## Patentansprüche

1. Stimulator- und lontophoresevorrichtung (A), die zur Behandlung von Netzhaut- und Sehnerverkrankungen verwendet wird, umfassend:
- eine aus zwei Teilen bestehende Kappe (10), wobei eine die vordere Kappe (10a) ist, die eine elektromagnetische Spulenbaugruppe (12) umfasst, die elektromagnetische Spulen (121), die 4 elektromagnetische Felder in einem Hornhautscheitelabstand zu den Augäpfeln erzeugen, und Kühlkäfige (122) enthält, wobei jede elektromagnetische Spule (121) mit einem entsprechenden Kühlkäfig abgedeckt ist, und die andere die hintere Kappe (10b) ist, die ebenfalls eine elektromagnetische Spulenbaugruppe (12) umfasst, die elektromagnetische Spulen (121), die 4 elektromagnetische Felder, die den okzipitalen Kortexbereich stimulieren, und 1 elektromagnetisches Feld, das den interhemisphärischen Bereich stimuliert, erzeugen, und Kühlkäfige (122) enthält, wobei jede elektromagnetische Spule (121) mit einem entsprechenden Kühlkäfig abgedeckt ist,
- die Vorrichtung zudem einen elektronischen Steuerkasten (20) umfasst, der Folgendes umfasst: einen Kühlkompressor (22), einen Perfluorkohlenstoff-Gastank (23), eine elektronische Karte und einen Prozessor (21), der einen Zähler (211) darauf aufweist, der so konfiguriert ist, dass er die Intensität, Frequenz und Zeit des von den elektromagnetischen Spulen (121) erzeugten elektromagnetischen Feldes einstellt, und einen Lautsprecher (25), der mit der elektronischen Karte und dem Prozessor (21) verbunden ist, wobei der Kühlkompressor (22) über Kühlrohre (124), die in einem Verbindungsrohr (30) untergebracht sind, mit den Kühlkäfigen (122) im Inneren der vorderen Kappe (10a) und der hinteren Kappe (10b) verbunden ist.

2. Stimulator- und lontophoresevorrichtung (A) nach Anspruch 1, umfassend eine Verbindungsschiene (11), die sich an den Seiten der vorderen Kappe (10a) und der hinteren Kappe (10b) befindet, um die vordere Kappe (10a) und die hintere Kappe (10b) miteinander zu verbinden, sodass der Abstand zwischen der vorderen und der hinteren Kappe verlängert oder verkürzt wird.

3. Stimulator- und lontophoresevorrichtung (A) nach Anspruch 1, wobei die vordere Kappe (10a) und die hintere Kappe (10b) aus nicht entflammbarem, antibakteriellem, antistatischem medizinischem Kunststoff oder Kohlefaser hergestellt sind.

4. Stimulator- und lontophoresevorrichtung (A) nach Anspruch 1, wobei die Abschnitte neben der elektromagnetischen Spulenbaugruppe (12) an den Innenflächen der vorderen Kappe (10a) und der hinteren Kappe (10b) mit einem intelligenten Moosgummi (13) bedeckt sind.

5. Stimulator- und lontophoresevorrichtung (A) nach Anspruch 4, wobei der Moosgummi (13) aus einem medizinischen, entflammbaren, biokompatiblen, antiallergischen und antibakteriellen Material hergestellt ist.

6. Stimulator- und lontophoresevorrichtung (A) nach Anspruch 1, wobei sich die elektromagnetische Spule (121) im Inneren der elektromagnetischen Spulenbaugruppe (12) innerhalb der vorderen Kappe (10a) und der hinteren Kappe (10b) befindet und eine polygonale Form aufweist und so konfiguriert ist, dass sie über 30 Minuten ein 2000-Milligauss-Magnetfeld einer Frequenz von 42 Hertz erzeugt.

7. Stimulator- und lontophoresevorrichtung (A) nach Anspruch 1 oder 6, wobei der Kühlkäfig (122), der sich auf der elektromagnetischen Spule (121) befindet, so konfiguriert ist, dass er das Risiko einer thermischen Schädigung der Augäpfel auf zellulärer Ebene verhindert.

8. Stimulator- und lontophoresevorrichtung (A) nach Anspruch 1 oder 7, wobei der Kühlkäfig (122) ein doppellagiger, undurchlässiger Hohlkasten aus Glasfaser, Kohlefaser oder einem anderen medizinischen Material ist.

9. Stimulator- und lontophoresevorrichtung (A) nach Anspruch 1 oder 7, wobei der Kühlkäfig (122) einen Kühltank (1221), der eine Gaszirkulation darin ermöglicht, und eine Gasauslassöffnung (1222) und eine Gaseinlassöffnung (1223), die den Eintritt und Austritt des Kühlgases ermöglichen, aufweist.

10. Stimulator- und lontophoresevorrichtung (A) nach Anspruch 1, wobei die Kühlrohre (124) so konfiguriert sind, dass sie eine Rezirkulation zwischen dem Kühltank (1221) und dem in dem Kühlkäfig (122) enthaltenen Kühlkompressor ermöglichen.

11. Stimulator- und lontophoresevorrichtung (A) nach Anspruch 1, umfassend ein Bedienfeld (26), das sich in dem elektronischen Steuerkasten (20) befindet und es ermöglicht, die Intensität, Frequenz und Zeit sowie den Aktiv-Passiv-Zustand der elektromagnetischen Spulen (121) über USB- oder WIRELESS-Verbindungen einzustellen und gleichzeitig den Kühlgrad des Kühlgases in den Kühlkäfigen (122) einzustellen.

12. Stimulator- und lontophoresevorrichtung (A) nach Anspruch 1, wobei der Zähler (211), der sich in dem elektronischen Steuerkasten (20) befindet, so konfiguriert ist, dass er es dem den Patienten betreuenden Arzt ermöglicht, die Behandlungskompatibilität des Patienten und die Häufigkeit der Verwendung der Stimulator- und lontophoresevorrichtung (A) durch den Patienten zu überwachen.

13. Stimulator- und lontophoresevorrichtung (A) nach Anspruch 1, wobei der Lautsprecher (25), der mit der elektronischen Karte und dem Prozessor (21) verbunden ist, es ermöglicht, den Patienten mit Audioanweisungen in verschiedenen Sprachen zu leiten.

14. Stimulator- und lontophoresevorrichtung (A) nach Anspruch 1 oder 6, umfassend eine LED-Anzeige (1211), die sich auf der elektromagnetischen Spule (121) befindet und es ermöglicht, die aktiven und passiven elektromagnetischen Spulen (121) rückzuverfolgen.

15. Stimulator- und lontophoresevorrichtung (A) nach Anspruch 1 oder 6, umfassend magnetische Sensoren (1212), die sich auf der elektromagnetischen Spule (121) befinden und das Magnetfeld, das erzeugt wird, wenn die elektromagnetische Spule (121) aktiv ist, auf den Bildschirm (24), der in dem elektronischen Steuerkasten (20) enthalten ist, reflektieren und gleichzeitig bestimmen, ob sich die elektromagnetischen Spulenbaugruppen (12) im richtigen symmetrischen Kontaktabstand zu dem Patienten befinden.

16. Stimulator- und lontophoresevorrichtung (A) nach Anspruch 1, umfassend einen Bildschirm (24), der sich auf dem elektronischen Steuerkasten (20) befindet und über den Zähler (211) die gesammelten Informationen, wie z. B. die Magnetfeldstärke der elektromagnetischen Spulen (121), die Frequenz, die Zeit und die elektromagnetische Spule (121), die aktiv ist, die Anzahl der Verwendungen und die Aktivierungsinformationen wiedergibt.

17. Stimulator- und lontophoresevorrichtung (A) nach Anspruch 1, wobei das Verbindungsrohr (30) zwischen der Kappe (10) und dem elektronischen Steuerkasten (20) positioniert ist und als Gruppe die elektrischen Kabel (123), die zu der elektromagnetischen Spule (121) führen und aus dem elektronischen Steuerkasten (20) austreten, und die Kühlrohre (124) zwischen dem Kühlkompressor (22) und dem Kühlkäfig (122) aufnimmt, sodass sie isoliert und separat sind.

18. Stimulator- und lontophoresevorrichtung (A) nach Anspruch 1, umfassend ein Netzteil (27), das sich im Inneren des elektronischen Steuerkastens (20) befindet und ein(e) direkt mit dem Stromnetz arbeitende(r) Batterie-Akku und/oder Adapter ist, wobei das Netzteil über elektrische Kabel (123) mit den elektromagnetischen Spulen (121) verbunden ist.

## Revendications

1. Dispositif de stimulation et d'iontophorèse (A) utilisé pour traiter les maladies de la rétine et du nerf optique, comprenant :
- un capuchon (10) constitué de deux parties ; l'une étant le capuchon avant (10a) comprenant un ensemble de bobines électromagnétiques (12) incluant des bobines électromagnétiques (121) qui créent 4 champs électromagnétiques à une distance vertex des globes oculaires, et des cages de refroidissement (122), chaque bobine électromagnétique (121) étant recouverte d'une cage de refroidissement respective, et l'autre étant le capuchon arrière (10b) comprenant également un ensemble de bobines électromagnétiques (12) incluant des bobines électromagnétiques (121) qui créent 4 champs électromagnétiques qui stimulent la région du cortex occipital et 1 champ électromagnétique qui stimule la région interhémisphérique, et des cages de refroidissement (122), chaque bobine électromagnétique (121) étant recouverte d'une cage de refroidissement respective,
- le dispositif comprenant également un boîtier de commande électronique (20), comprenant : un compresseur de refroidissement (22), un réservoir de perfluorocarbone gazeux (23), une carte électronique et un processeur (21) ayant un compteur (211) conçu sur celui-ci pour régler l'intensité, la fréquence et le temps du champ électromagnétique créé par les bobines électromagnétiques (121), et un haut-parleur (25) connecté à la carte électronique et au processeur (21), dans lequel le compresseur de refroidissement (22) est connecté aux cages de refroidissement (122) à l'intérieur du capuchon avant (10a) et du capuchon arrière (10b) par le biais de tuyaux de refroidissement (124) qui sont logés dans un tuyau de connexion (30).

2. Dispositif de stimulation et d'iontophorèse (A) selon la revendication 1, comprenant un rail de connexion (11) qui est situé au niveau des côtés du capuchon avant (10a) et du capuchon arrière (10b) afin de connecter le capuchon avant (10a) et le capuchon arrière (10b) l'un à l'autre de sorte à allonger ou raccourcir la distance entre le capuchon avant et le capuchon arrière.

3. Dispositif de stimulation et d'iontophorèse (A) selon la revendication 1, dans lequel le capuchon avant (10a) et le capuchon arrière (10b) sont constitués d'une fibre plastique ou de carbone médicale ininflammable, antibactérienne, antistatique.

4. Dispositif de stimulation et d'iontophorèse (A) selon la revendication 1, dans lequel les sections en plus de l'ensemble de bobines électromagnétiques (12) au niveau des surfaces internes du capuchon avant (10a) et du capuchon arrière (10b), sont recouvertes d'un caoutchouc spongieux (13) intelligent.

5. Dispositif de stimulation et d'iontophorèse (A) selon la revendication 4, dans lequel ledit caoutchouc spongieux (13) est constitué d'un matériau médical, inflammable, biocompatible, antiallergique et antibactérien.

6. Dispositif de stimulation et d'iontophorèse (A) selon la revendication 1, dans lequel la bobine électromagnétique (121) est située à l'intérieur de l'ensemble de bobines électromagnétiques (12) au sein desdits capuchon avant (10a) et capuchon arrière (10b) et a une forme polygonale et est conçue pour créer un champ magnétique de 2000 milligauss pendant 30 minutes à une fréquence de 42 hertz.

7. Dispositif de stimulation et d'iontophorèse (A) selon la revendication 1 ou 6, dans lequel ladite cage de refroidissement (122) située sur ladite bobine électromagnétique (121), est conçue pour prévenir le risque de dommage thermique se produisant aux niveaux cellulaires sur les globes oculaires.

8. Dispositif de stimulation et d'iontophorèse (A) selon la revendication 1 ou 7, dans lequel ladite cage de refroidissement (122) est une boîte creuse imperméable à double couche en fibre de verre, fibre de carbone ou un quelconque autre matériau médical.

9. Dispositif de stimulation et d'iontophorèse (A) selon la revendication 1 ou 7, dans lequel ladite cage de refroidissement (122) a un réservoir de refroidissement (1221) qui permet la circulation de gaz dans celui-ci, et un orifice de sortie de gaz (1222) et un orifice d'entrée de gaz (1223) permettant l'entrée et l'évacuation du gaz de refroidissement.

10. Dispositif de stimulation et d'iontophorèse (A) selon la revendication 1, dans lequel lesdits tuyaux de refroidissement (124) sont conçus pour permettre la recirculation entre le réservoir de refroidissement (1221) et le compresseur de refroidissement inclus dans la cage de refroidissement (122).

11. Dispositif de stimulation et d'iontophorèse (A) selon la revendication 1, comprenant un panneau de commande (26) situé dans le boîtier de commande électronique (20) qui permet de régler l'intensité, la fréquence, le temps et l'état actif-passif des bobines électromagnétiques (121) par le biais de connexions USB ou SANS FIL et en même temps, permet de régler le degré de refroidissement du gaz de refroidissement dans les cages de refroidissement (122).

12. Dispositif de stimulation et d'iontophorèse (A) selon la revendication 1, dans lequel ledit compteur (211) situé dans le boîtier de commande électronique (20) est conçu pour permettre au médecin qui suit le patient de surveiller la compatibilité de traitement du patient et la fréquence d'utilisation du stimulateur et du dispositif d'iontophorèse (A) par le patient.

13. Dispositif de stimulation et d'iontophorèse (A) selon la revendication 1, dans lequel ledit haut-parleur (25) connecté à ladite carte électronique et audit processeur (21), permet de guider le patient à l'aide de commandes audio dans différentes langues.

14. Dispositif de stimulation et d'iontophorèse (A) selon la revendication 1 ou 6, comprenant un indicateur LED (1211) situé sur ladite bobine électromagnétique (121) qui permet de suivre les bobines électromagnétiques (121) actives et passives.

15. Dispositif de stimulation et d'iontophorèse (A) selon la revendication 1 ou 6, comprenant des capteurs magnétiques (1212) situés sur la bobine électromagnétique (121) qui reflètent le champ magnétique créé lorsque la bobine électromagnétique (121) est active, sur l'écran (24) inclus dans le boîtier de commande électronique (20) et en même temps qui déterminent si les ensembles de bobines électromagnétiques (12) sont à la bonne distance de contact symétrique par rapport au patient.

16. Dispositif de stimulation et d'iontophorèse (A) selon la revendication 1 comprenant un écran (24) situé sur le boîtier de commande électronique (20), qui reflète par le biais du compteur (211) les informations collectées, telles que l'intensité de champ magnétique des bobines électromagnétiques (121), la fréquence, le temps et la bobine électromagnétique (121) qui est active, le nombre d'utilisations, et les informations d'activation.

17. Dispositif de stimulation et d'iontophorèse (A) selon la revendication 1, dans lequel ledit tuyau de connexion (30) est positionné entre le capuchon (10) et le boîtier de commande électronique (20) et se loge en tant que groupe, les câbles électriques (123) menant à la bobine électromagnétique (121) et sortant du boîtier de commande électronique (20) et les tuyaux de refroidissement (124) entre le compresseur de refroidissement (22) et la cage de refroidissement (122), de telle sorte qu'ils sont isothermes et isolés.

18. Dispositif de stimulation et d'iontophorèse (A) selon la revendication 1, comprenant une alimentation électrique (27) située à l'intérieur du boîtier de commande électronique (20), qui est un accumulateur de batterie et/ou un adaptateur fonctionnant directement avec le réseau électrique, dans lequel ladite alimentation électrique est connectée aux bobines électromagnétiques (121) par le biais de câbles électriques (123).
